Europäisches Patentamt

European Patent Office

Office européen des brevets

(11) Numéro de publication : **0 342 139 B1**

# (12) FASCICULE DE BREVET EUROPEEN

(45) Date de publication du fascicule du brevet :
**31.03.93 Bulletin 93/13**

(51) Int. Cl.$^5$ : **A61K 31/195**

(21) Numéro de dépôt : **89450007.3**

(22) Date de dépôt : **05.04.89**

(54) **Utilisation de l'Aspartate d'Arginine pour la fabricament d'un médicament pour traiter certains troubles de la mémoire.**

(30) Priorité : **09.05.88 US 191391**

(43) Date de publication de la demande :
**15.11.89 Bulletin 89/46**

(45) Mention de la délivrance du brevet :
**31.03.93 Bulletin 93/13**

(84) Etats contractants désignés :
**BE CH DE ES FR GB IT LI LU NL**

(56) Documents cités :
**FR-A- 106**
**PRENSA MED. ARGENT, vol. 71, no. 15, 1984; E.E.VALDES, pp. 708-711***
**ANN. PHARM. FRANCAISES, vol. 42, no. 3, 1984, Masson, Paris (FR); F.GUINARD et al., pp. 265-270***

(56) Documents cités :
**CHEMICAL ABSTRACTS, vol. 92, no. 3, 21 janvier 1980, Columbus, OH (US); p. 201, J.POEY et al., no. 16913k, and J.ELSAIR et al., no. 16914m***
**MERCK MANUAL, 15th ed., R.Berkow, MD, 1987, Merck Sharp & Dohme Research Lab., Rahway, NJ (US)***

(73) Titulaire : **CORTIAL S.A.**
**7 rue de l'Armorique**
**F-75015 Paris (FR)**

(72) Inventeur : **Cehovic, Georges**
**7 Rue La Vallée**
**F-91120 Palaiseau (FR)**
Inventeur : **Panconi, Emmanuel**
**Rue du Maréchal Gallieni**
**F-33700 Mérignac (FR)**

(74) Mandataire : **Phélip, Bruno et al**
**c/o Cabinet Harlé & Phélip 21, rue de La Rochefoucauld**
**F-75009 Paris (FR)**

## Description

La présente invention concerne un médicament pour traiter les troubles de la mémoire comme par exemple ceux induits par : le vieillissement , la fatigue autre que la fatigue psychique, la consommation chronique d'alcool,les traitements par électrochoc et les médicaments tels que les benzodiazépines comme le diazépam et les cholinolytiques comme la scopolamine. Le traitement est assuré par l'administration réitérée d'aspartate d'arginine.

L'aspartate d'arginine est un produit décrit en France dans les brevets spéciaux de médicaments n° 1625 M et 106 CAM ainsi que dans le brevet FR 2 568124 Ce produit est le sel résultant de la réaction en quantités équimoléculaires de l'acide L-aspartique sur la L-arginine.

On a décrit dans ces documents les effets physiologiques et un mode d'action de ce produit qui le fait utiliser en thérapeutique dans :

* les états hyperammoniémiques,
* l'asthénie psychique et physique,
* et certains retards de croissance.

On a de plus déjà décrit dans un article de Valdes et Debian ( Prensa Med. Argent, 71: 708,1984) l'utilisation de compositions contenant de la vincamine , de l'acide ascorbique et optionnellement de l'aspartate d'arginine dans le traitement d'insuffisance cérébro-vasculaire chronique. Dans cet article , l'aspartate d'arginine n'est pas utilisée seul mais obligatoirement en combinaison.

Deux résumés des Chemical Abstracts sont relatifs à l'utilisation de l'aspartate d'arginine à l'encontre de l'alcoolisme chronique et aigu. Le résumé 92 16913k ( volume 92 , page 201, 1980) concerne l'action de l'aspartate d'arginine sur les troubles métaboliques et hépatiques induits par la consommation d'alcool chez les rats.

Le résumé 92 16 914 m (volume 92, page 201, 1980) concerne quant à lui l'effet de l'aspartate d'arginine sur la stimulation de l'oxydation de l'éthanol chez l'homme . Il est indiqué que le traitement par l'aspartate d'arginine diminue les effets subjectifs de l'alcoolisme aigu.

Il a été trouvé maintenant d'une façon tout à fait surprenante que l'aspartate d'arginine, lorsqu'il est utilisé par voie orale pouvait être utile dans certains troubles de la mémoire.

La présente invention a donc pour objet l'utilisation de l'aspartate d'arginine comme ingrédient actif pour la fabrication d'un médicament pour le traitement des troubles de mémoire autres que ceux induits par l'asthénie psychique et l'alcoolisme aigu.

Préférentiellement , on administre par voie orale une dose journalière d'aspartate d'arginine. Avantageusement, cette dose est administrée de façon réitérée et est comprise entre 100 et 1000 mg/kg/j.

Ce médicament peut être présenté sous une forme d'ampoule buvable , de sirop, de sachet ou de comprimé.

Les électrochocs sont une thérapeutique très utile dans le traitement de patients atteints de psychose majeure et de troubles importants de la personalité. La thérapeutique par les électrochocs présente néanmoins des effets secondaires importants, troubles de la mémoire, bien que les modalités d'application actuellement utilisées entrainent un peu moins d'effets secondaires. Pour une revue sur la thérapeutique par les électrochocs voir l'article de SELVIN-B-L, Electroconvulsive therapy-1987, Anesthesiology, 1987, 67, 367-385.

Les benzodiazépines et les cholinolytiques sont des médicaments très utilisés mais qui entrainent souvent une perte de mémoire : GOODMAN and GILMAN, The pharmacological basis of therapeutics, 7ième édition, page 341-342, 133.

La consommation d'alcool chez l'homme entraine des lésions cérébrales associées à des déficits d'apprentissage et de mémoire, décrites dans le syndrome de KORSAKOFF, pour plus de détail voir The Merck Manual 15 ième édition page 1330.

Il vient d'être démontré maintenant, que l'aspartate d'arginine utilisé par voie orale, dans divers modèles d'amnésie induite chez le rat ou la souris, diminue de façon importante la perte de mémoire.

## RESULTATS PHARMACOLOGIQUES

La mise en évidence chez l'animal de l'activité de l'aspartate d'arginine sur les processus mnésiques s'est faite à l'aide du test d'évitement passif et du test d'apprentissage en labyrinthe en T.

Le test d'évitement passif, est réalisé dans un appareillage comportant deux compartiments, l'un clair, l'autre obscur. Un animal est placé dans la chambre claire. Lorsque l'animal passe dans la chambre obscure un choc electrique de 0,3 mA lui est administré jusqu'à son retour dans la chambre claire, c'est la phase d'apprentissage. 24 heures plus tard l'animal est replacé dans la chambre claire et l'on mesure le temps de latence pour passer dans la chambre obscure c'est la phase d'épreuve. Une augmentation de ce temps de latence reflète la mémorisation par l'animal du choc électrique reçu la veille. L'administration d'un traitement amnésiant avant ou après la phase d'apprentissage entraine une réduction du temps de latence de sortie. L'efficacité d'un traitement anti amnésiant est mesurée par sa capacité à s'opposer à cette diminution du temps de latence de sortie.

Trois traitements amnésiants ont été utilisés pour étudier l'effet de l'aspartate d'arginine à l'aide du test d'évitement passif. Il s'agit de l'amnésie induite par l'électrochoc chez le rat, celle induite par la scopolamine chez la souris et celle induite par le diazépam chez la souris.

Dans le test d'amnésie induite par l'électrochoc deux groupes de 10 rats ont été utilisés. Un groupe a été traité par l'aspartate d'arginine, par voie orale et à la dose de 50mg/kg par jour pendant 7 jours. Un groupe d'animaux contrôle n'ont re-u que de l'eau, solvant de l'aspartate d'arginine. Au septième jours de l'essai tous les animaux ont reçu un électrochoc ( 100 volts, 0,4 seconde ) immédiatement après la phase d'apprentissage du test d'évitement passif. Les temps de latence de sortie lors de la phase d'épreuve 24 heures après la phase d'apprentissage sont présentés dans le tableau suivant.

| Traitement | Electrochoc | Temps de latence de sortie en secondes | Test de Fisher |
|---|---|---|---|
| Eau (animaux témoins) | 100 volts 0,4 seconde | 34,1 +/- 16,4 | |
| Aspartate d'arginine voie orale 500mg/kg/j | 100 volts 0,4 seconde | 104,1 +/- 11,4 | 3,69** |

** comparé aux témoins électrochoc

Les résultats montrent que les animaux prétraités par l'aspartate d'arginine ont un temps de latence de sortie significativement supérieur aux animaux du lot contrôle.

Dans le test d'amnésie induite par la scopolamine chez la souris selon le protocole du test d'évitement passif decrit par GLICK-S-D, ZIMMERBERG-B, Behavorial Biology 1972, 7, 245-54, l'aspartate d'arginine a été administré par voie orale à la dose de 2048mg/kg deux fois par jour pendant 3,5 jours. L'amnésie a été induite par l'injection intrapéritonéale de scopolamine 1 mg/kg 30 minutes avant la phase d'apprentissage. La dernière administration d'aspartate d'arginine est effectuée 60 minutes avant la phase d'apprentissage. Les expériences ont été effectuées en aveugle avec 20 souris par groupe expérimental. Le tableau suivant donne les résultats obtenus dans le test d'évitement passif en comparaison avec le piracetam substance de référence nootrope administré en dose unique 2048 mg/kg par voie orale 30 minutes avant l'agent amnésiant.

| Traitement | Scopolamine voie ip 30 minutes avant l'apprentissage | Temps de latence de sortie en secondes | Test de Student |
|---|---|---|---|
| Eau (animaux témoins) | 0 | 175,9 +/-3,9 | |
| Eau (animaux témoins amnésie) | 1 mg/kg | 123,7 +/- 11,1 | 4,543* |
| Aspartate d'arginine Voie orale 2048 mg/kg 7 administrations | 1 mg/kg | 162,9 +/- 6,9 | 3,091** |
| Piracetam 2048 mg/kg Voie orale 1 administration | 1 mg/kg | 171,5 +/- 4,2 | 4,139** |

* comparé aux témoins sans scopolamine
** comparé aux temoins avec scopolamine

Ces résultats montrent que le traitement par l'aspartate d'arginine permet aux souris de résister à l'effet amnésiant de la scopolamine et que la protection apportée par l'aspartate d'arginine est comparable à celle du piracetam.

Dans le test d'amnésie induite par le diazepam chez la souris l'aspartate d'arginine a été administré par voie orale, en aigu, à la dose de 2048 mg/kg. L'amnésie a été induite par l'injection intrapéritonéale de diazepam 1 mg/kg 30 minutes avant la phase d'apprentissage. Les expériences ont été effectuées en aveugle avec 20 souris par groupe expérimental. Le tableau suivant donne les résultats obtenus dans le test d'évitement passif en comparaison avec le piracetam substance de référence nootrope administré en dose unique 2048 mg/kg par voie orale 30 minutes avant l'agent amnésiant.

| Traitement | Diazépam voie ip 30 minutes avant l'apprentissage | Temps de latence de sortie en secondes | Test de student |
|---|---|---|---|
| Eau (animaux témoins) | 0 | 175,3 +/- 3,3 | |
| Eau (animaux témoins) amnésie | 1 mg/kg | 116,0 +/- 15,2 | 3,923* |
| Aspartate d'arginine Voie orale 2048mg/kg 1 administration | 1 mg/kg | 157,1 +/- 12,6 | 2,135** |
| Piracetam Voie orale 2048 mg/kg 1 administration | 1 mg/kg | 173,1 +/- 3,9 | 3,738** |

\* comparé aux témoins sans diazepam

\*\* comparé aux temoins avec diazepam

Ces résultats montrent que l'administrastion aigue d'aspartate d'arginine ou de piracetam permet de réduire l'effet amnésiant du diazepam.

L'étude de l'activité de l'aspartate d'arginine vis à vis du déficit de mémoire induit par une consommation chronique d'alcool a été évaluée par une épreuve de mémoire dite d'alternance différée au moyen d'un labyrinthe en T. Les animaux utilisés sont des souris mâles de souche Balb/c agées de 14 mois. Elles sont réparties en trois groupes de 10, 9 et 9 animaux. Le premier groupe de dix souris constitue le groupe témoin, ces animaux n'ont pas consommé d'alcool. Les deuxième et troisième groupes d'animaux ont consommé, comme boisson pendant 12 mois, une solution aqueuse d'éthanol à 12% (v/v) et ont été sevrés 3 à 5 semaines avant l'expérience. Le deuxième groupe constitue le groupe traité par un placebo. Le troisième groupe reçoit de l'aspartate d'arginine, par voie orale à la dose de 3000 mg/kg par jour pendant toute la durée de l'épreuve c'est à dire pendant 10 jours.

L'épreuve est effectuée dans un labyrinthe en T. Chaque souris est soumise quotidiennement pendant 6 jours à 6 essais séparés par un interval de 30 secondes. Au cours de chaque essai, l'animal choisit librement entre les deux bras (droit ou gauche) du labyrinthe et il y reste enfermer pendant 30 secondes. Il est alors replacé au départ pour un nouvel essai. L'animal choisi dans l'essai n un bras du labyrhinte, si dans l'essai n+1 l'animal choisi le même bras l'alternance est nulle, si au contraire l'animal choisi l'autre bras l'alternance est positive, il y a donc 5 alternances pour les 6 essais. L'alternance est comptabilisée par rapport à l'essai n. Les résultats repris dans le tableau suivant sont exprimés en pourcentage d'alternances positives par groupes de souris et cumulés sur les six jours de l'expérience c'est à dire sommes des alternances réussies par le groupe (X) de souris chaque jour pour l'essai n+1 par rapport à l'essai n divisé par le nombre total des alternances par essai pour le groupe X, ce nombre est de 60 pour le groupe 1, et est de 54 pour les groupes 2 et 3.

| Numéro de l'alternance | 1 | 2 | 3 | 4 | 5 |
|---|---|---|---|---|---|
| Traitement | | | | | |
| Eau (témoins) | 82 | 65 | 72 | 75 | 73 |
| Eau (témoins alcooliques) | 76 | 57 | 52* | 54* | 57* |
| Aspartate d'arginine voie orale 3000 mg/kg 10 jours d'administration | 82 | 69 | 73** | 75** | 66 |

\* $p<0,01$ par rapport au groupe témoin eau

\*\* $p<0,01$ par rapport au groupe témoin alcoolique

Ces résultats prouvent qu'un traitement chronique par l'aspartate d'arginine des animaux alcooliques entraine une récupération totale du potentiel mnésique des animaux.

De ces quatre tests pharmacologiques on peut conclure que l'administration réitérée d'aspartate d'arginine prévient de façon évidente les troubles de la mémorisation induits par l'application de traitements réputés amnésiants.

Les modèles pharmacologiques d'amnésie utilisés dans nos recherches sont classiquement utilisés pour l'étude des composés nootropes tels que le piracetam, qui administré à l'homme agé améliore significativement ses troubles de mémoire, ( HALTMAN-H-J, NORMILE-H-J, GERSHON-S, Non-cholinergic pharmacology in human cognitive disorders, in Cognitive Neurochemistry, edited by STAHL-S-M, IVERSEN-S-D, GOODMAN-E-C, 1987, Oxford Science Publications). Ainsi les résultats obtenus sur ces tests sont prédictifs d'une activité de l'aspartate d'arginine vis à vis des troubles de mémoires consécutifs au vieillissement.

Ces activités pharmacologiques de l'aspartate d'arginine sur une fonction spécifique du système nerveux central sont surprenantes compte tenu des connaissances acquises jusqu'alors sur les propriétés de cette substance, à savoir la stimulation de l'anabolisme protéique.

## RESULTATS TOXICOLOGIQUES

L'administration par voie orale de différentes doses d'aspartate d'arginine à des rats Sprague Dawley ou à des souris Swiss a montré que les doses létales à 50% (DL50) sont très élevées chez l'une ou l'autre des deux espèces, et supérieures à 20 g/kg de poids corporel.

Compte tenu, de sa très faible toxicité et des activités pharmacologiques de l'aspartate d'arginine vis à vis de l'amnésie induite par les électrochocs, la scopolamine, le diazépam et la consommation chronique d'alcool, l'aspartate d'arginine constitue un traitement de choix des pathologies mnésiques.

## Revendications

1. Utilisation de l'aspartate d'arginine pour la fabrication d'un médicament pour le traitement des troubles de la mémoire autres que ceux induits par l'asthémie psychique et l'alcoolisme aigu.

2. Utilisation selon la revendication 1,caractérisée en ce que l'on administre par voie orale une dose journalière d'aspartate d'arginine.

**3.** Utilisation selon la revendication 2, caractérisée en ce que ladite dose est administrée de façon réitérée, et est comprise entre 100 et 1000 mg/kg/jour .

**4.** Utilisation selon l'une des revendications 2 et 3 , caractérisée en ce que ladite dose est présentée sous forme d'ampoules buvables , de sirop , de sachets ou de comprimés .

**5.** Utilisation selon l'une des revendications 1 à 4 pour la fabrication d'un médicament pour le traitement des troubles de la mémoire induits par le vieillissement , la fatigue autre que la fatigue psychique, les benzodiazépines , les cholinolytiques, les électrochocs ou ceux résultants de la consommation chronique d'alcool .

**Patentansprüche**

**1.** Verwendung von Argininaspartat zur Herstellung eines Medikaments zur Behandlung von Gedächtnisstörungen außer denen, die durch psychische Asthenie und akute Alkoholvergiftung hervorgerufen werden.

**2.** Verwendung nach Anspruch 1, dadurch gekennzeichnet, daß eine tägliche Dosis Argininaspartat oral verabreicht wird.

**3.** Verwendung nach Anspruch 2, dadurch gekennzeichnet, daß die Dosis wiederholt verabreicht wird und zwischen 100 und 1000 mg/kg/Tag liegt.

**4.** Verwendung nach einem der Ansprüche 2 und 3, dadurch gekennzeichnet, daß die Dosis in Form von Trinkampullen, Sirup, Pulverbriefchen oder Tabletten dargereicht wird.

**5.** Verwendung nach einem der Ansprüche 1 bis 4 zur Herstellung eines Medikaments zur Behandlung von Gedächtnisstörungen, hervorgerufen durch Altern, Müdigkeit außer psychischer Müdigkeit, Benzodiazepine, Cholinolytika, Elektroschocks oder solchen, die aus chronischem Alkoholkonsum resultieren.

**Claims**

**1.** Use of arginine aspartate for making a medicine for the treatment of memory disturbances , except those induced by psychic asthenia and acute alcoholism .

**2.** Use according to claim 1 , characterized in that a daily dose of arginine aspartate is administered orally .

**3.** Use according to claim 2, characterized in that the said dose is administered repeatedly , and is comprised between 100 and 1000 mg/kg/day.

**4.** Use according to one of claims 2 and 3 , characterized in that the said dose is under the form of drinkable phials , syrup , bags or tablets.

**5.** Use according to one of claims 1 to 4 for making a medecine for the treatment of memory disturbances caused by ageing , tiredness , except psychic tiredness , benzodiazepines , cholinolytics, electroshocks or those resulting from the chronical consumption of alcohol .